(19) Europäisches Patentamt
European Patent Office
Office européen des brevets

(11) Veröffentlichungsnummer: **0 148 393 A1**


# (12) EUROPÄISCHE PATENTANMELDUNG


(21) Anmeldenummer: **84114311.8**

(22) Anmeldetag: **27.11.84**

(51) Int. Cl.⁴: **C 07 C 103/37**, C 07 C 103/38, C 07 C 119/06, A 01 N 37/18

(30) Priorität: **07.12.83 DE 3344201**

(43) Veröffentlichungstag der Anmeldung: **17.07.85 Patentblatt 85/29**

(84) Benannte Vertragsstaaten: **AT BE CH DE FR GB IT LI NL SE**



(71) Anmelder: **BAYER AG, Konzernverwaltung RP Patentabteilung, D-5090 Leverkusen 1 Bayerwerk (DE)**

(72) Erfinder: **Heinemann, Ulrich, Dr., Feldstrasse 18, D-5653 Leichlingen (DE)**
Erfinder: **Thomas, Rudolf, Dr., Dellbusch 269, D-5600 Wuppertal 2 (DE)**
Erfinder: **Santel, Hans-Joachim, Dr., Gerstenkamp 19, D-5000 Koeln 80 (DE)**
Erfinder: **Schmidt, Robert R., Dr., Im Waldwinkel 110, D-5060 Bergisch-Gladbach 2 (DE)**
Erfinder: **Eue, Ludwig, Dr., Paul-Klee-Strasse 36, D-5090 Leverkusen 1 (DE)**


(54) **N-Chloracetyl-enamine.**


(57) Neue N-Chloracetyl-enamine der Formel

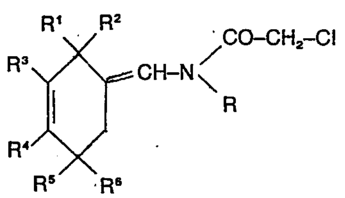

(I)

in welcher

R für Alkyl, Alkenyl, Alkinyl, Alkoxyalkyl, Dialkoxyalkyl, Alkylthioalkyl oder Halogenalkyl steht und

$R^1$, $R^2$, $R^3$, $R^4$, $R^5$ und $R^6$ unabhängig voneinander für Wasserstoff, Halogen, Alkyl, Alkenyl, Alkinyl, Alkoxy, Alkanoyloxy, Dialkylamino oder für gegebenenfalls substituiertes Aryl stehen, und außerdem

$R^1$ und $R^3$ oder

$R^1$ und $R^5$ und/oder

$R^3$ und $R^4$ oder

$R^4$ und $R^5$ jeweils gemeinsam für eine gegebenenfalls substituierte Alkylen-, Cycloalkylen-, Alkenylen-, Arylen- oder Etherbrücke, oder für eine Sauerstoffbrücke stehen können,

ein Verfahren zur Herstellung der neuen Stoffe und deren Verwendung als Herbizide.


EP 0 148 393 A1

BAYER AKTIENGESELLSCHAFT 5090 Leverkusen, Bayerwerk
Konzernverwaltung RP
Patentabteilung Dü/Ke-c
Ia

N-Chloracetyl-enamine

Die Erfindung betrifft neue N-Chloracetyl-enamine, ein Verfahren zu ihrer Herstellung und ihre Verwendung als Herbizide.

Es ist bereits bekannt, daß bestimmte Chloracetamide und N-Chloracetyl-enamine herbizide Eigenschaften besitzen (vgl. DE-OS 23 28 340 und DE-OS 31 20 990). So lassen sich zum Beispiel das 2-Chlor-N-(2-ethyl-6-methyl-phenyl)-N-(1-methoxy-prop-2-yl)-acetamid und das N-(4-Cyclohex-1-enyliden-metheno)-2,6-dimethyl-chloracetanilid zur Bekämpfung von Unkraut verwenden. Die Wirkung dieser bekannten Verbindungen ist jedoch, insbesondere bei niedrigen Aufwandmengen und Konzentrationen, nicht immer in allen Anwendungsbereichen völlig zufriedenstellend.

Es wurden nun neue N-Chloracetyl-enamine der allgemeinen Formel (I),

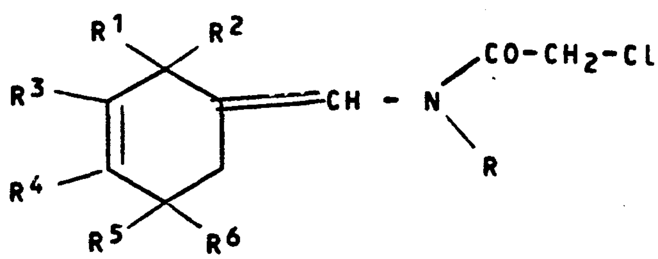

(I)

in welcher

R für Alkyl, Alkenyl, Alkinyl, Alkoxyalkyl, Dialkoxyalkyl, Alkylthioalkyl oder Halogenalkyl steht und

$R^1$, $R^2$, $R^3$, $R^4$, $R^5$ und $R^6$ unabhängig voneinander für Wasserstoff, Halogen, Alkyl, Alkenyl, Alkinyl, Alkoxy, Alkanoyloxy, Dialkylamino oder für gegebenenfalls substituiertes Aryl stehen und außerdem

$R^1$ und $R^3$ oder
$R^1$ und $R^5$ und/oder
$R^3$ und $R^4$ oder
$R^4$ und $R^5$ jeweils gemeinsam für eine gegebenenfalls substituierte Alkylen-, Cycloalkylen-, Alkenylen-, Arylen-, oder Etherbrücke, oder für eine Sauerstoffbrücke stehen können, gefunden.

Weiterhin wurde gefunden, daß man die neuen N-Chloracetyl-enamine der Formel (I) erhält, wenn man Aldehyde der Formel (II),

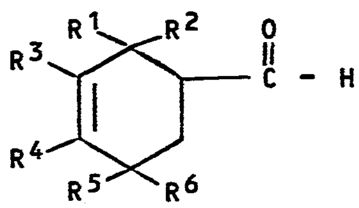

(II)

in welcher

$R^1$, $R^2$, $R^3$, $R^4$, $R^5$ und $R^6$ die oben angegebene Bedeutung haben,

zunächst in einer ersten Stufe mit Aminen der Formel (III),

$$R - NH_2 \qquad (III)$$

in welcher

R die oben angegebene Bedeutung hat,

gegebenenfalls in Gegenwart eines Verdünnungsmittels und gegebenenfalls in Gegenwart eines Katalysators umsetzt und die so erhaltenen Azomethine der Formel (IV),

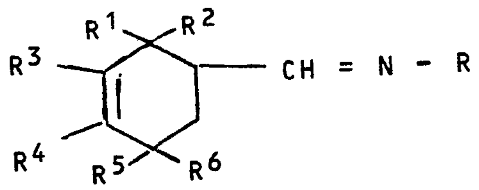

(IV)

in welcher

R, $R^1$, $R^2$, $R^3$, $R^4$, $R^5$ und $R^6$ die oben angegebene Bedeutung haben,

in einer 2. Stufe mit Chloracetylchlorid, gegebenenfalls in Gegenwart eines Verdünnungsmittels und gegebenenfalls in Gegenwart eines Säurebindemittels umsetzt.

Schließlich wurde gefunden, daß die neuen N-Chloracetyl-enamine der Formel (I) herbizide Eigenschaften besitzen.

Überraschenderweise zeigen die neuen N-Chloracetyl-enamine der Formel (I) eine bessere herbizide Wirksamkeit als das 2-Chlor-N-(2-ethyl-6-methyl-phenyl)-N-(1-methoxy-prop-2-yl)-acetamid und das N-(4-Cyclohex-1-enyliden-metheno)-2,6-dimethyl-chloracetanilid, welches konstitutionell ähnliche, vorbekannte Verbindungen gleicher Wirkungsrichtung sind.

Die erfindungsgemäßen Stoffe sind durch die Formel (I) allgemein definiert. In dieser Formel steht

R vorzugsweise für geradkettiges oder verzweigtes Alkyl mit 1 bis 10 Kohlenstoffatomen, Alkenyl mit 2 bis 10 Kohlenstoffatomen, Alkinyl mit 2 bis 10 Kohlenstoffatomen, geradkettiges oder verzweigtes Alkoxyalkyl mit 1 bis 6 Kohlenstoffatomen in der Alkoxygruppe und 1 bis 6 Kohlenstoffatomen im Alkylteil, Dialkoxyalkyl mit 1 bis 6 Kohlenstoffatomen in jeder Alkoxygruppe und 1 bis 6 Kohlenstoffatomen im Alkylteil, Alkylthioalkyl mit 1 bis 6 Kohlenstoffatomen im Alkylthioteil und 1 bis 6 Kohlenstoffatomen im Alkylteil oder für geradkettiges oder verzweigtes Halogenalkyl mit 1 bis 6 Kohlenstoffatomen und 1 bis 9 gleichen oder verschiedenen Halogenatomen und

$R^1$, $R^2$, $R^3$, $R^4$, $R^5$ und $R^6$ stehen unabhängig voneinander vorzugsweise für Wasserstoff, Fluor, Chlor, Brom, Jod, geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Alkoxy mit 1 bis 4 Kohlenstoffatomen, Alkanoyloxy mit 1 bis 4 Kohlenstoffatomen in der Alkan-Gruppe, Dialkylamino mit 1 bis 4 Kohlenstoffatomen in jeder Alkyl-Gruppe oder für gegebenenfalls durch Halogen, Cyano, Nitro, Alkyl mit 1 bis 4 Kohlenstoffatomen und/oder Alkoxy mit 1 bis 4 Kohlenstoffatomen substituiertes Aryl mit 6 bis 10 Kohlenstoffatomen, und außerdem stehen

$R^1$ und $R^3$ oder
$R^1$ und $R^5$ und/oder
$R^3$ und $R^4$ oder
$R^4$ und $R^5$ jeweils gemeinsam vorzugsweise für eine gegebenenfalls durch Halogen, Alkyl mit 1 bis 4 Kohlenstoffatomen und/oder Alkoxy mit 1 bis 4 Kohlenstoffatomen substituierte Alkylenbrücke mit 1 bis 6 Kohlenstoffatomen, eine gegebenenfalls durch Halogen, Alkyl mit 1 bis 4 Kohlenstoffatomen und/oder Alkoxy mit 1 bis 4 Kohlenstoffatomen substituierte Cycloalkylenbrücke mit 4 bis 8 Kohlenstoffatomen, für eine gegebenenfalls durch Halogen, Alkyl mit 1 bis 4 Kohlenstoffatomen und/oder Alkoxy mit 1 bis 4 Kohlenstoffatomen substituierte Alkenylenbrücke mit 2 bis 6 Kohlenstoffatomen, eine gegebenenfalls durch Halogen, Alkyl mit 1 bis 4 Kohlenstoffatomen und/oder Alkoxy mit 1 bis

4 Kohlenstoffatomen substituierte Arylenbrücke mit 6 bis 10 Kohlenstoffatomen oder für eine gegebenenfalls durch Halogen, Alkyl mit 1 bis 4 Kohlenstoffatomen und/oder Alkoxy mit 1 bis 4 Kohlenstoffatomen substituierte Etherbrücke mit 2 bis 6 Kohlenstoffatomen, oder für eine Sauerstoffbrücke.

Besonders bevorzugt sind N-Chloracetyl-enamine der Formel (I), in denen

R für Methyl, Ethyl, n- und i-Propyl, n-, i-, s- und t-Butyl, Allyl, Butenyl, Propargyl, Methoxymethyl, Methoxyethyl, Dimethoxyethyl, Ethoxyethyl, Methylthiomethyl, Methylthioethyl, Ethylthioethyl, Chlormethyl, Dichlormethyl, Trichlormethyl oder Trichlorethyl steht und

$R^1$, $R^2$, $R^3$, $R^4$, $R^5$ und $R^6$ unabhängig voneinander für Wasserstoff, Fluor, Chlor, Brom, Methyl, Ethyl, n- und i-Propyl, n-, i-, s- und t-Butyl, n- und i-Pentyl, n- und i-Hexyl, Allyl, n- und i-Butenyl, n- und i-Pentenyl, n- und i-Hexenyl, Propargyl, n- und i-Butinyl, Methoxy, Ethoxy, n- und i-Propoxy, n- und i-Butoxy, Acetoxy, Propionyloxy, Dimethylamino, Diethylamino, Dipropylamino, Dibutylamino oder für gegebenenfalls ein- bis dreifach, gleich oder verschieden durch Fluor, Chlor, Brom, Nitro, Cyano, Methyl, Ethyl, Methoxy oder Ethoxy substituiertes Phenyl oder Naphthyl stehen, und außerdem

$R^1$ und $R^3$ oder
$R^1$ und $R^5$ und/oder
$R^3$ und $R^4$ oder
$R^4$ und $R^5$ jeweils gemeinsam für eine gegebenenfalls ein- bis dreifach, gleich oder verschieden durch Chlor, Brom, Methyl, Methoxy, Ethyl, Ethoxy, Propyl, Propoxy, Butyl und/oder Butoxy substituierte Alkylenbrücke mit 1 bis 4 Kohlenstoffatomen, für eine gegebenenfalls ein- bis dreifach, gleich oder verschieden durch Chlor, Brom, Methyl, Methoxy, Ethyl, Ethoxy, Propyl, Propoxy, Butyl und/oder Butoxy substituierte Cycloalkylenbrücke der Formel

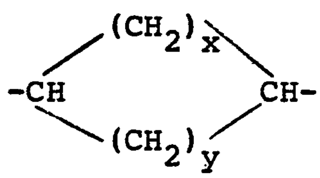

stehen, in welcher
x und y unabhängig voneinander für 1, 2 oder 3 stehen,

oder für eine gegebenenfalls ein- bis dreifach, gleich oder verschieden durch Chlor, Brom, Methyl, Methoxy, Ethyl, Ethoxy, Propyl, Propoxy, Butyl und/oder Butoxy substituierte Alkenylenbrücke mit 2 bis 4 Kohlenstoffatomen und einer oder zwei Doppelbindungen, für eine gegebenenfalls ein- bis dreifach, gleich oder verschieden durch Chlor, Brom, Methyl, Methoxy, Ethyl, Ethoxy, Propyl, Propoxy, Butyl und/oder Butoxy substituierte

ortho-Phenylenbrücke oder für eine gegebenenfalls ein- bis dreifach, gleich oder verschieden durch Chlor, Brom, Methyl, Methoxy, Ethyl, Ethoxy, Propyl, Propoxy, Butyl und/oder Butoxy substituierte Etherbrücke der Formel

$$-(CH_2)_m-O-(CH_2)_n-$$

stehen, in welcher
m und n unabhängig voneinander für 1 oder 2 stehen, oder für eine Sauerstoffbrücke stehen.

Im einzelnen seien außer den bei den Herstellungsbeispielen genannten Verbindungen die folgenden N-Chloracetyl-enamine der allgemeinen Formel (I) genannt:

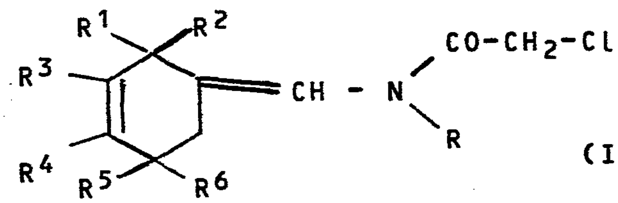

(I)

Tabelle 1

| R | 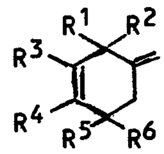 |
|---|---|
| $CH_3$ | 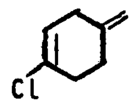 |
| $C_2H_5$ | 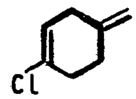 |
| $(CH_3)_2CH-$ | 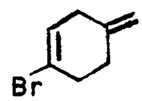 |
| $CH_3O-(CH_2)_2-$ | 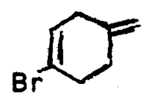 |
| $(CH_3O)_2CH-CH_2-$ | 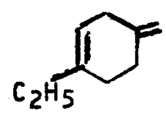 |
| $CH_3-(CH_2)_2-$ | 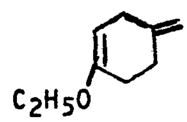 |
| $CH_3-(CH_2)_3-$ | 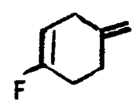 |
| $CH_3$ | 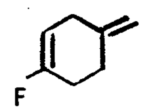 |
| $C_2H_5$ | 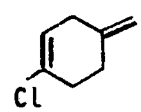 |
| $(CH_3)_2CH-$ | 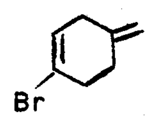 |
| $CH_3-(CH_2)_2-$ | 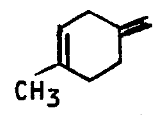 |

**Tabelle 1** (Fortsetzung)

| R | 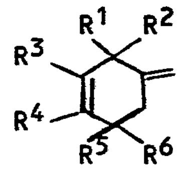 |
|---|---|
| $CH_3-(CH_2)_3-$ | 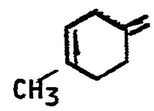 |
| $(CH_3)_2CH-CH_2-$ | 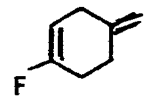 |
| $CH_3-CH(C_2H_5)-$ | 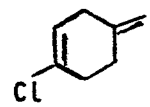 |
| $(CH_3)_3C-$ | 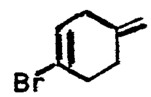 |
| $CH_3O-CH_2-$ | 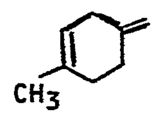 |
| $CH_3O-(CH_2)_2-$ | 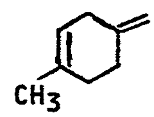 |
| $(CH_3O)_2CH-CH_2-$ | 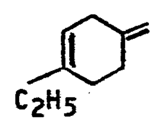 |
| $CH_2=CH-CH_2-$ | 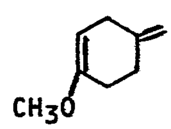 |
| $CH_3$ | 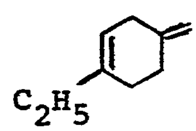 |
| $C_2H_5$ | 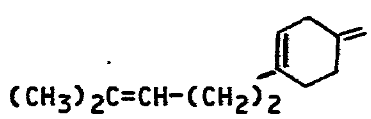 |

Tabelle 1 (Fortsetzung)

| R | 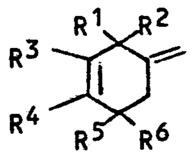 |
| --- | --- |
| $(CH_3)_2CH-$ | 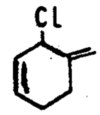 |
| $CH_3-(CH_2)_2-$ | 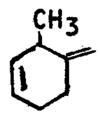 |
| $CH_3-(CH_2)_3-$ | 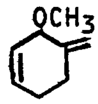 |
| $(CH_3)_2CH-CH_2-$ | 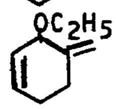 |
| $CH_3-CH(C_2H_5)-$ | 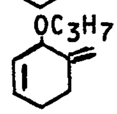 |
| $(CH_3)_3C-$ | 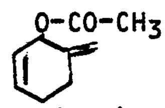 |
| $CH_3O-CH_2-$ | 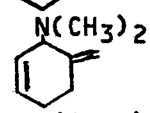 |
| $CH_3O-(CH_2)_2-$ | 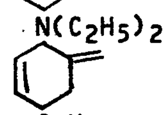 |
| $(CH_3O)_2CH-CH_2-$ | 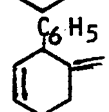 |
| $CH_2=CH-CH_2-$ | 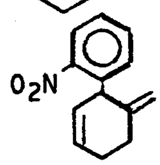 |
| $CH_3$ | 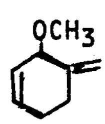 |
| $C_2H_5$ | 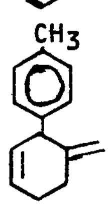 |

Tabelle 1 (Fortsetzung)

| R | 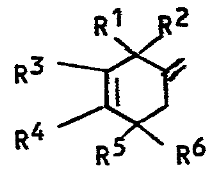 |
|---|---|
| $(CH_3)_2CH-$ | 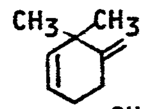 |
| $CH_3-(CH_2)_2-$ | 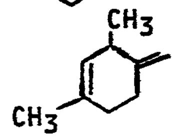 |
| $CH_3-(CH_2)_3-$ | 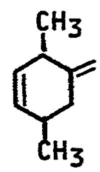 |
| $(CH_3)_2CH-CH_2-$ | 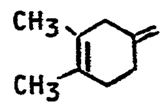 |
| $CH_3-CH(C_2H_5)-$ | 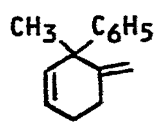 |
| $(CH_3)_3C-$ | 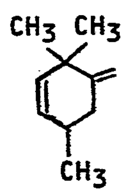 |
| $CH_3O-CH_2-$ | 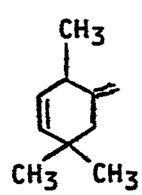 |
| $CH_3O-(CH_2)_2-$ | 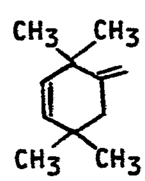 |

Tabelle 1 (Fortsetzung)

| R | 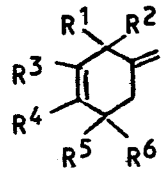 |
|---|---|
| $(CH_3O)_2CH-CH_2-$ | 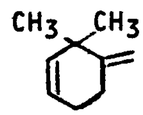 |
| $CH_2=CH-CH_2-$ | 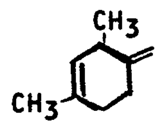 |
| $CH_3$ | 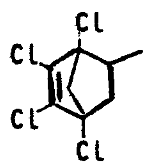 |
| $C_2H_5$ | 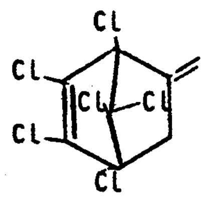 |
| $(CH_3)_2CH-$ | 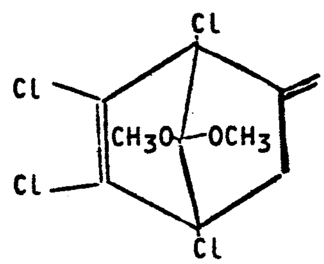 |
| $CH_3-(CH_2)_2-$ | 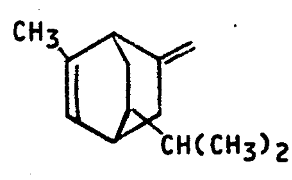 |
| $CH_3-(CH_2)_3-$ | 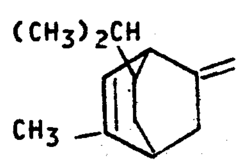 |

**Tabelle 1** (Fortsetzung)

| R | 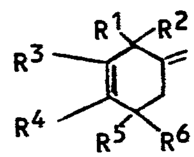 |
|---|---|
| $(CH_3)_2CH-CH_2-$ | 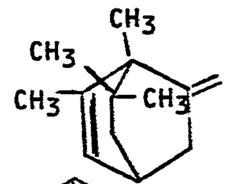 |
| $CH_3-CH(C_2H_5)-$ | 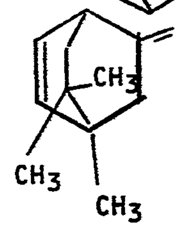 |
| $(CH_3)_3C-$ | 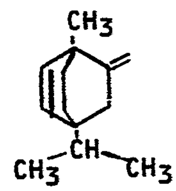 |
| $CH_3O-CH_2-$ | 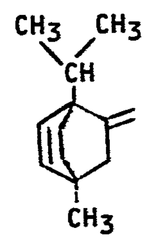 |
| $CH_3O-(CH_2)_2-$ | 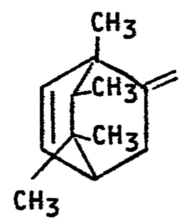 |
| $(CH_3O)_2CH-CH_2-$ | 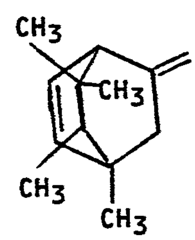 |

Tabelle 1 (Fortsetzung)

| R | 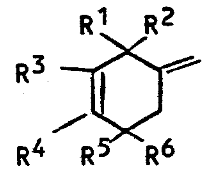 |
|---|---|
| $CH_2=CH-CH_2-$ | 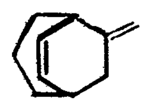 |
| $CH_3$ | 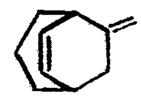 |
| $C_2H_5$ | 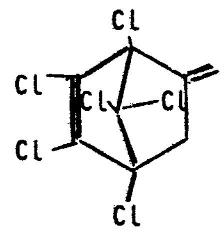 |
| $(CH_3)_2CH-$ | 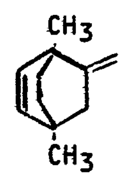 |
| $CH_3-(CH_2)_2-$ | 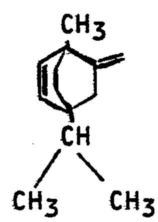 |
| $CH_3-(CH_2)_3-$ | 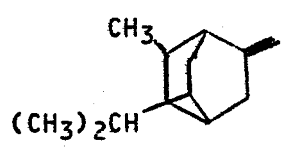 |
| $(CH_3)_2CH-CH_2-$ | 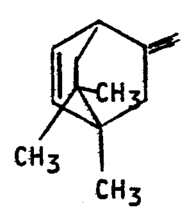 |
| $CH_3-CH(C_2H_5)-$ | 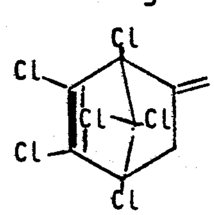 |

**Tabelle 1** (Fortsetzung)

| R | 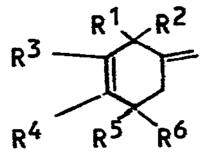 |
|---|---|
| $(CH_3)_3C-$ | 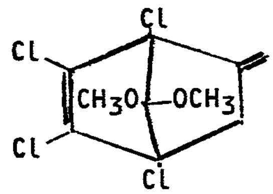 |
| $CH_3O-CH_2-$ | 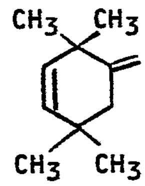 |
| $CH_3O-(CH_2)_2-$ | 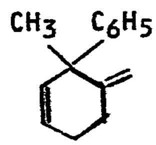 |
| $(CH_3O)_2CH-CH_2-$ | 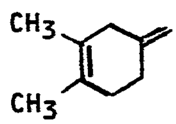 |
| $CH_2=CH-CH_2-$ | 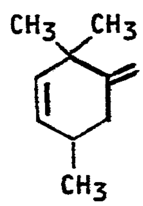 |
| $CH_3$ | 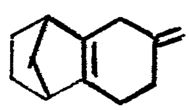 |
| $C_2H_5$ | 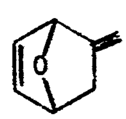 |
| $(CH_3)_2CH-$ | 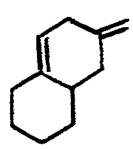 |

**Tabelle 1** (Fortsetzung)

| R | 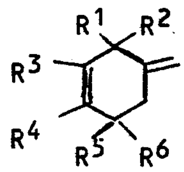 |
|---|---|
| $CH_3-(CH_2)_2-$ | 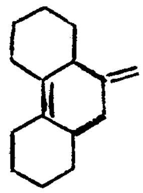 |
| $CH_3-(CH_2)_3-$ | 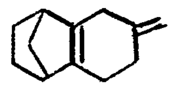 |
| $(CH_3)_2CH-CH_2-$ | 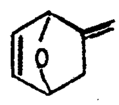 |
| $CH_3-CH(C_2H_5)-$ | 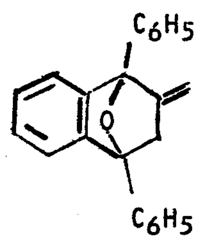 |
| $(CH_3)_3C-$ | 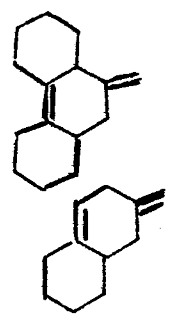 |
| $CH_3O-CH_2-$ | |
| $CH_3O-(CH_2)_2-$ | 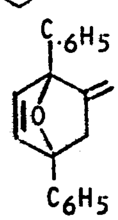 |
| $(CH_3O)_2CH-CH_2-$ | 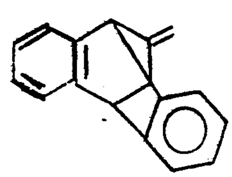 |

<u>Tabelle 1</u> (Fortsetzung)

| R | 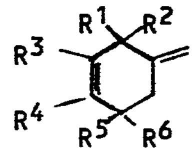 |
|---|---|
| $CH_2$=CH-$CH_2$- | 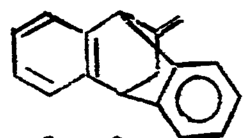 |
| $CH_3$ | 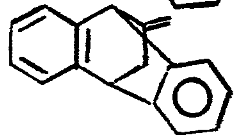 |
| $C_2H_5$ | 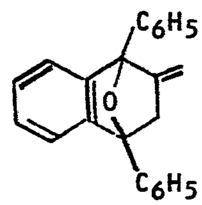 |
| $CH_3$-$(CH_2)_2$- | 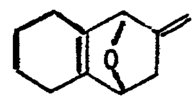 |
| $CH_3$-$(CH_2)_3$- | 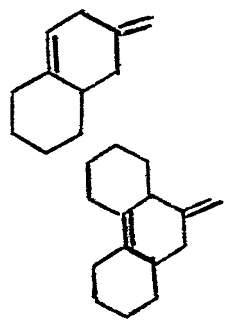 |
| $(CH_3)_2$CH-$CH_2$- | |
| $CH_3$-CH($C_2H_5$)- | 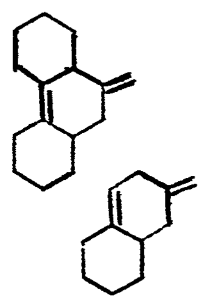 |
| $(CH_3)_3$C- | |
| $CH_3$O-$CH_2$- | 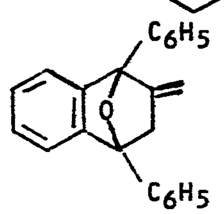 |

Tabelle 1 (Fortsetzung)

| R | 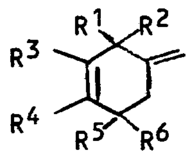 |
|---|---|
| $CH_3O-(CH_2)_2-$ | 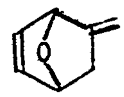 |
| $(CH_3O)_2CH-CH_2-$ | 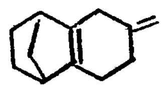 |
| $CH_2=CH-CH_2-$ | 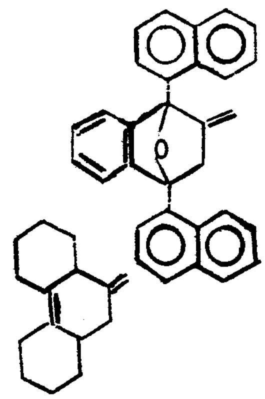 |
| $(CH_3)_2CH-$ | |

Verwendet man 5-Norbornen-2-aldehyd und Isopropylamin als Ausgangsstoffe und Chloracetylchlorid als weitere Reaktionskomponente, so läßt sich der Verlauf des erfindungsgemäßen Verfahrens durch das folgende Formelschema veranschaulichen:

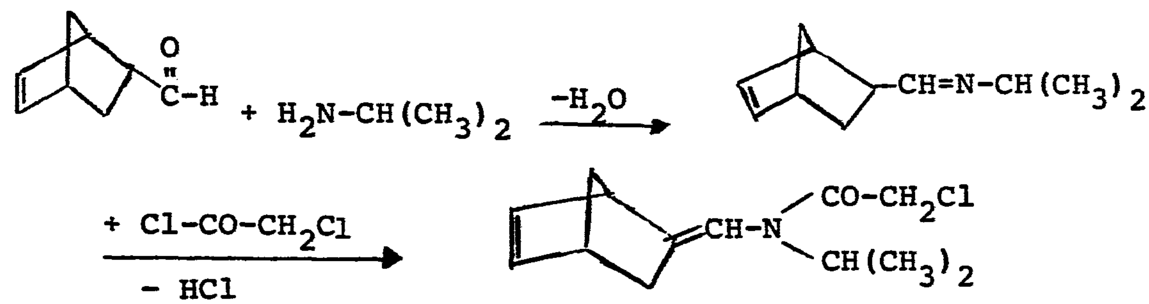


Die zur Durchführung des erfindungsgemäßen Verfahrens als Ausgangsstoffe benötigten Aldehyde sind durch die Formel (II) allgemein definiert. In dieser Formel (II) stehen $R^1$, $R^2$, $R^3$, $R^4$, $R^5$ und $R^6$ vorzugsweise für diejenigen Reste, die schon bei der Beschreibung der erfindungsgemäßen Stoffe der Formel (I) als bevorzugt für diese Substituenten angegeben wurden. Die Aldehyde der Formel (II) sind bekannt. (Vergl. z.B. J.Amer.chem. Soc. 98, 1992-1993 [1976]; C.W. Smith "Acrolein", J.Wiley and Sons, New York 1962; S. 216, 218)

Die weiterhin zur Durchführung des erfindungsgemäßen Verfahrens als Ausgangsstoffe benötigten Amine sind durch die Formel (III) allgemein definiert. In dieser Formel (III) steht R vorzugsweise für diejenigen Reste, die schon bei der Beschreibung der erfindungsgemäßen Stoffe

der Formel (I) als bevorzugt für diesen Substituenten genannt wurden. Die Amine der Formel (III) sind allgemein bekannte Verbindungen der organischen Chemie.

Das bei der Durchführung des erfindungsgemäßen Verfahrens in der zweiten Stufe als Reaktionskomponente benötigte Chloracetylchlorid ist bekannt.

Als Verdünnungsmittel kommen bei der Durchführung der ersten Stufe des erfindungsgemäßen Verfahrens inerte organische Lösungsmittel infrage. Vorzugsweise verwendbar sind aromatische Kohlenwasserstoffe, wie Benzol, Toluol und Xylol.

Als Katalysatoren können bei der Durchführung der ersten Stufe des erfindungsgemäßen Verfahrens alle üblicherweise für derartige Kondensationen verwendbaren Reaktionsbeschleuniger eingesetzt werden. Vorzugsweise verwendbar sind saure Katalysatoren, wie beispielsweise p-Toluolsulfonsäure.

Die Reaktionstemperaturen können bei der Durchführung der ersten Stufe des erfindungsgemäßen Verfahrens innerhalb eines größeren Bereiches variiert werden. Im allgemeinen arbeitet man bei Temperaturen zwischen 50°C und 280°C, vorzugsweise bei der Rückflußtemperatur des jeweils verwendeten Verdünnungsmittels.

Bei der Durchführung der ersten Stufe des erfindungsgemäßen Verfahrens arbeitet man im allgemeinen unter Normaldruck.

Zur Durchführung der ersten Stufe des erfindungsgemäßen Verfahrens setzt man pro Mol Aldehyd der Formel (II) 1 bis 10 Mol, vorzugsweise 1 bis 2 Mol an Amin der Formel (III) ein. Bei gasförmigen Aminen ist es möglich, diese gelöst in einem geeigneten Lösungsmittel, wie beispielsweise Wasser einzusetzen. Es ist jedoch auch möglich, einen kontinuierlichen Gasstrom durch das Reaktionsgefäß zu leiten. Die Aufarbeitung und Isolierung der Azomethine der Formel (IV) geschieht nach allgemein üblichen Verfahren.

Als Verdünnungsmittel kommen bei der Durchführung der zweiten Stufe des erfindungsgemäßen Verfahrens alle inerten organischen Lösungsmittel in Betracht. Vorzugsweise verwendbar sind aliphatische oder aromatische, gegebenenfalls halogenierte Kohlenwasserstoffe, wie Benzin, Benzol, Toluol, Chlorbenzol, Chloroform, Dichlormethan, Tetrachlorkohlenstoff, Ether wie Diethylether, Dioxan, Tetrahydrofuran oder Ethylenglykoldimethylether, Ketone, wie Aceton oder Butanon, Nitrile, wie Acetonitril oder Propionitril, Ester, wie Essigsäureethylester oder die hochpolaren Lösungsmittel Dimethylformamid, Dimethylsulfoxid oder Hexamethylphosphorsäuretriamid.

Als Säurebindemittel kommen bei der Durchführung der zweiten Stufe des erfindungsgemäßen Verfahrens alle üblicherweise verwendbaren anorganischen und organischen Basen in Betracht. Vorzugsweise verwendet man tertiäre aliphatische oder aromatische Amine, wie Triethylamin, N,N-Dimethylanilin, 4-Dimethylaminopyridin, Pyridin, Diazabicyclononen (DBN) oder Diazabicycloundecen (DBU).

Die Temperaturen können bei der Durchführung der 2. Stufe des erfindungsgemäßen Verfahrens in einem größeren Bereich variiert werden. Im allgemeinen arbeitet man zwischen 20°C und 140°C, vorzugsweise zwischen 60°C und 100°C.

Bei der Durchführung der zweiten Stufe des erfindungsgemäßen Verfahrens arbeitet man ebenfalls im allgemeinen unter Normaldruck.

Zur Durchführung der 2. Stufe des erfindungsgemäßen Verfahrens setzt man pro Mol Azomethin der Formel (IV) im allgemeinen 1 bis 2 Mol, vorzugsweise 1 bis 1,2 Mol an Chloracetylchlorid und im allgemeinen 1 bis 2 Mol, vorzugsweise 1 bis 1,2 Mol an Säurebindemittel ein. Die Aufarbeitung und Isolierung der Reaktionsprodukte der Formel (I) erfolgt nach allgemein üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der

angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopercurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist

jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektriven Unkrautbekämpfung in einjährigen Kulturen eingesetzt werden.

Die erfindungsgemäßen Wirkstoffe zeigen neben einer sehr guten allgemein-herbiziden Wirksamkeit insbesondere auch eine hervorragende Verträglichkeit gegenüber wichtigen Kulturpflanzen. Sie können somit zur selektiven Unkrautbekämpfung sowohl in dikotylen Kulturen, wie beispielsweise Zuckerrüben, Raps, Kartoffeln, Soja, Baumwolle und Erbsen, ebenso wie in monokotylen Kulturen, wie Getreide, Reis oder Mais eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit

sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder

in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 4-Amino-6-(1,1-dimethylethyl)-3-ethylthio-1,2,4-triazin-5(4H)-on oder N-(2-Benzthiazolyl)-N,N'-dimethyl-harnstoff zur Unkrautbekämpfung in Getreide; 2-Chlor-4-ethylamino-6-isopropylamino-1,3,5-triazin zur Unkrautbekämpfung in Mais; 4-Amino-3-methyl-6-phenyl-1,2,4-trazin-5-(4H)-on zur Unkrautbekämfpung in Zuckerrüben und 4-Amino-6-(1,1-dimethylethyl)-3-methylthio-1,2,4-triazin-5(4H)-on zur Unkrautbekämpfung in Sojabohnen und Kartoffeln, infrage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele:

Beispiel 1

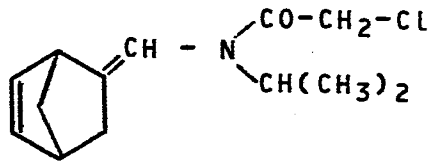

(I-1)

1. Stufe:

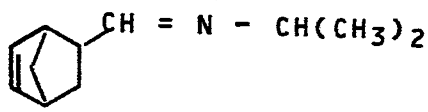

(IV-1)

47,5 g (0,39 Mol) 5-Norbornen-2-aldehyd und 32,9 g (0,39 Mol) 70 %ige wäßrige Isopropylaminlösung wurden in 200 ml Toluol unter Zusatz einer Spatelspitze p-Toluolsulfonsäure 6 Stunden am Wasserabscheider unter Rückfluß zum Sieden erhitzt. Die erhaltene Reaktionsmischung wurde im Vakuum eingeengt, und der Rückstand wurde im Wasserstrahlvakuum destilliert. Man erhielt 43,1 g (67 % der Theorie) an 2-Isopropyliminomethylen-5-norbornen vom Siedepunkt 77°C bis 79°C bei 19 mbar.

$^{1}$H-NMR-Spektrum($CDCl_3$): $\delta$ ppm = 0,9 - 1,3 (m, 10 H)
2,7 - 3,1 (m, 3 H)
3,2 (sept., 1 H, J=7 Hz)
6,1 (dd, 2 H, J=2 Hz)
7,6 (d, 1 H, J=6 Hz)

2. Stufe:

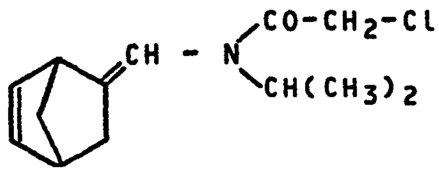

(I-1)

Zu 16,3 g (0,1 Mol) 2-Isopropyliminomethylen-5-norbornen und 8,0 g (0,1 Mol) Pyridin in 100 ml Tetrahydrofuran tropfte man bei Rückflußtemperatur 11,3 g (0,1 Mol) Chloracetylchlorid. Nach beendeter Zugabe erhitzte man weitere 60 Minuten auf Rückflußtemperatur. Zur Aufarbeitung wurde die erhaltene Reaktionsmischung in 500 ml eines Gemisches von Eis und Wasser gegossen und mit Dichlormethan extrahiert. Die vereinigten Dichlormethanphasen wurden 3 mal mit je 100 ml Wasser gewaschen, über Natriumsulfat getrocknet und im Vakuum eingeengt. Der Rückstand wurde einer Kugelrohrdestillation im Hochvakuum unterworfen. Man erhielt 18,5 g (77 % der Theorie) an 2-(N-Chloracetyl-N-isopropylaminomethanylyliden)-5-norbornen vom Siedepunkt 160°C bis 165°C bei 0,2 mbar.

$^1$H-NMR-Spektrum ($CDCl_3$): $\delta$(ppm) = 1,1 (d, 6 H, J=7 Hz),
1,4 - 2,1 (m, 4 H),
3,2 (m, 1 H),
3,4 (m, 1 H),
3,9 (s, 2 H),
4,6 (sept., 1 H, J=7 Hz)
und 6,0 - 6,3 (m, 3 H).

Nach der im Beispiel 1 angegebenen Methode wurden auch die in der folgenden Tabelle 2 formelmäßig aufgeführten Stoffe hergestellt.

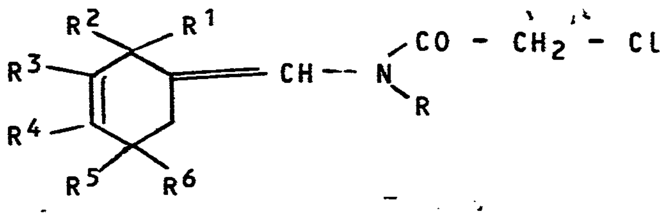

(I)

| Bsp.Nr. | Formel | physikal. Eigenschaften |
|---|---|---|
| 2 | 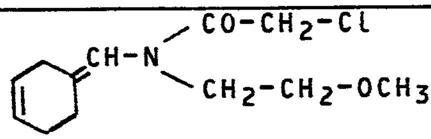 | Oel |
| 3 | 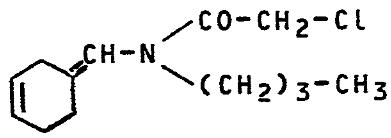 | $Kp_{0,2}$=162°C |
| 4 | 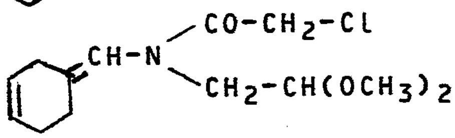 | $Kp_{0,6}$=161°C |
| 5 | 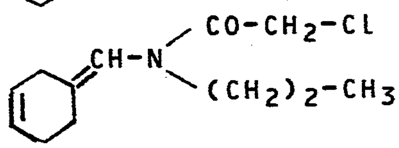 | $Kp_{0,2}$=150-160°C |
| 6 | 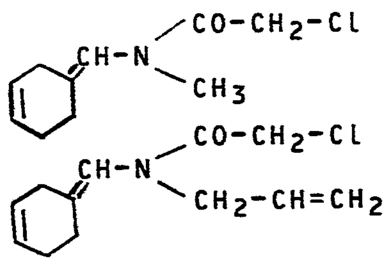 | $Kp_{0,2}$=120-125°C |
| 7 | | $Kp_{0,2}$=120-130°C |
| 8 | 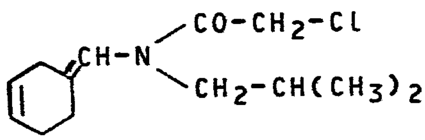 | $Kp_{0,5}$=160-165°C |
| 9 | 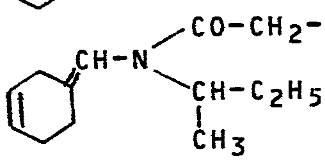 | $Kp_{0,9}$=170-175°C |
| 10 | 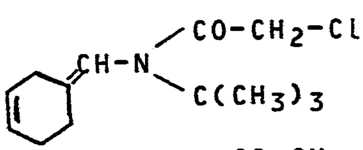 | $Kp_{0,5}$=165-170°C |
| 11 | 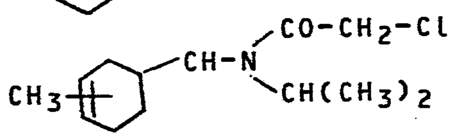 | Oel |

Tabelle 2 (Fortsetzung)

| Bsp.-Nr | Formel | physikal. Eigenschaften |
|---|---|---|
| 12 | 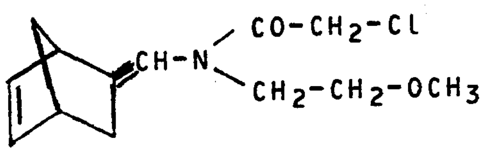 | $Kp_{0,15}$ = 170°C |
| 13 | 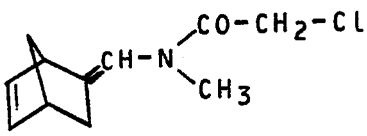 | $Kp_{0,3}$=150°C |
| 14 | 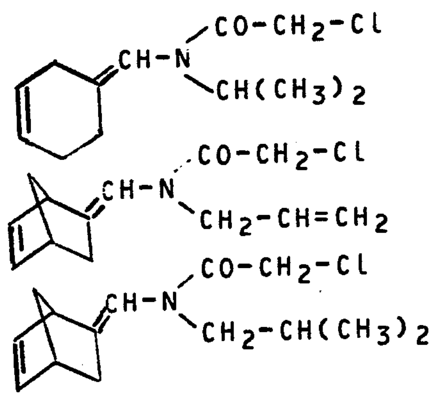 | $Kp_{0,2}$=160-165°C |
| 15 | | $Kp_{0,2}$=145-150°C |
| 16 | | $Kp_{0,2}$=135-140°C |
| 17 | 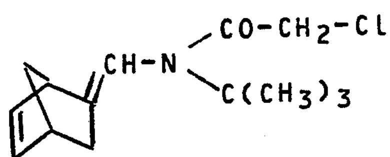 | $Kp_{0,3}$=165-170°C |
| 18 | 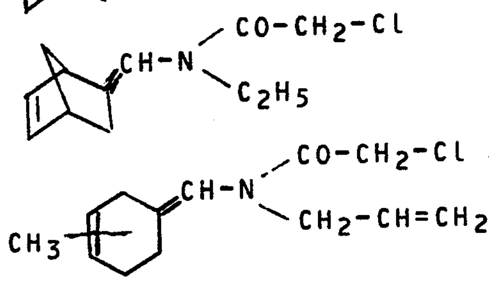 | $Kp_{0,2}$=155-160°C |
| 19 | | $Kp_{0,2}$=125-130°C |
| 20 | 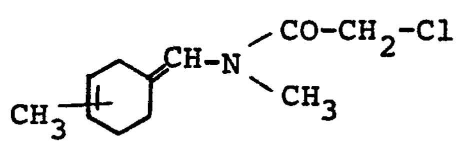 | $Kp_{0,1}$ = 95-100°C |
| 21 | 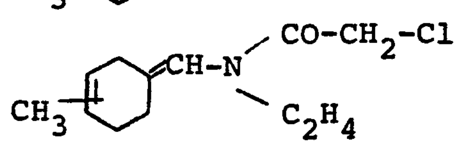 | $Kp_{0,1}$ = 125-130°C |

Tabelle 2: (Fortsetzung):

| Bsp. Nr. | Formel | physikalische Eigenschaften |
|---|---|---|
| 22 | 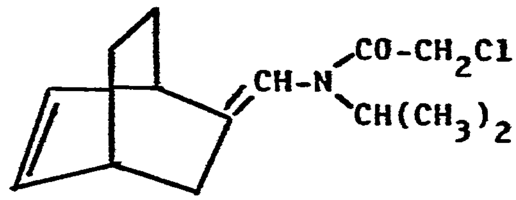 | Öl |
| 23 | 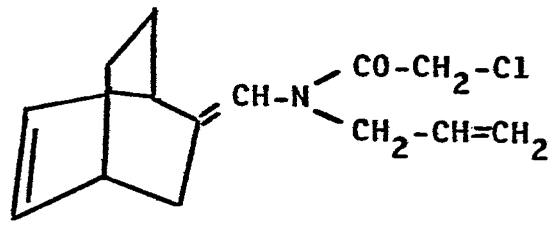 | $Kp_{0,2}$:128°C |
| 24 | 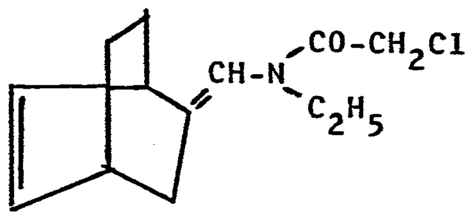 | $Kp_{0,2}$:130-134°C |
| 25 | 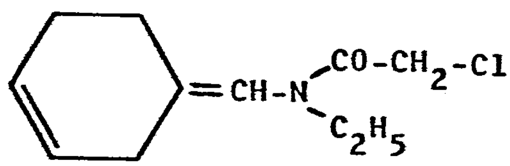 | $Kp_{0,4}$: 105-110°C |
| 26 | 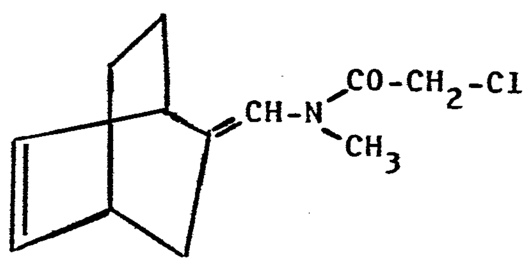 | $Kp_{0,5}$: 135-140°C |
| 27 | 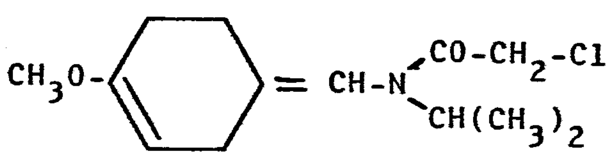 | Öl |

Nach der im Beispiel 1 angegebenen Methode wurden auch die in der folgenden Tabelle 3 formelmäßig aufgeführten Zwischenprodukte der Formel (IV) hergestellt:

Tabelle 3

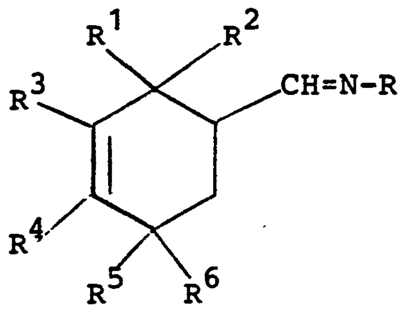

(IV)

| Bsp.-Nr. | Formel | physikalische Eigenschaften |
|---|---|---|
| IV-2 | 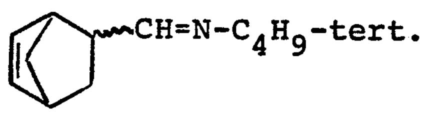 | Kp = 95-98°C/ 18 mbar |
| IV-3 | 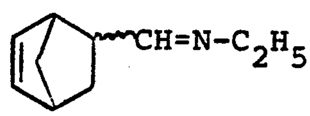 | Kp = 75-77°C/ 18 mbar |
| IV-4 | 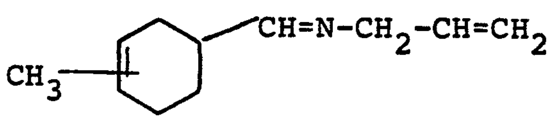 | Öl |
| IV-5 | 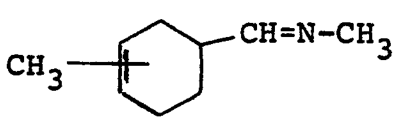 | Kp = 60-65°C/ 14 mbar |

Tabelle 3 (Fortsetzung)

| Bsp.-Nr. | Formel | physikalische Eigenschaften |
|---|---|---|
| IV-6 | 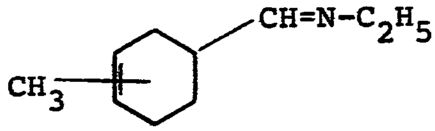 | Kp = 80-85°C/ 16 mbar |
| IV-7 | 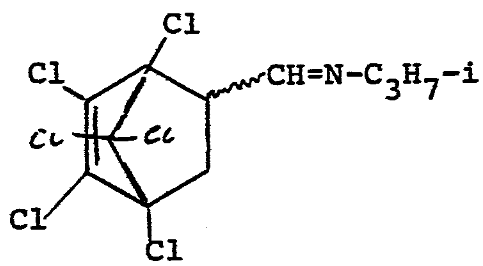 | Fp = 110-114°C |
| IV-8 | 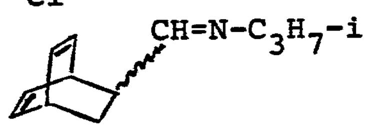 | Kp = 97-99°C/ 22 mbar |
| IV-9 | 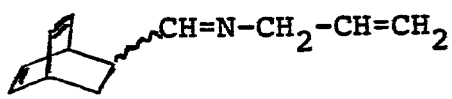 | Kp = 117°C/ 22 mbar |
| IV-10 | 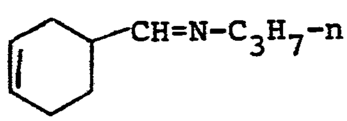 | Kp = 78-85°C/ 17 mbar |

Tabelle 3 (Fortsetzung)

| Bsp.-Nr. | Formel | physikalische Eigenschaften |
|---|---|---|
| IV-11 | 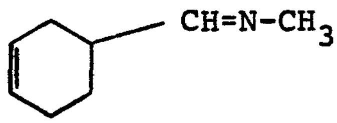 | Kp = 51-54°C/ 14 mbar |
| IV-12 | 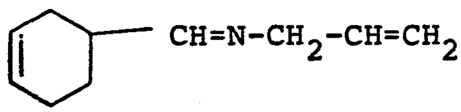 | Kp = 85°C/ 16 mbar |
| IV-13 | 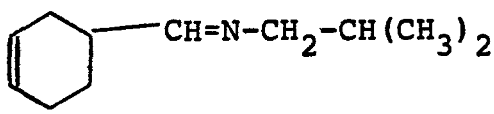 | Kp = 89-90°C/ 16 mbar |
| IV-14 | 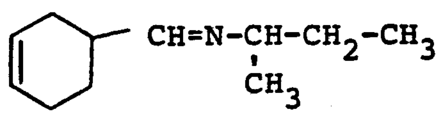 | Kp = 87-91°C/ 18 mbar |
| IV-15 | 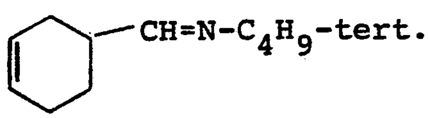 | Kp = 78-80°C/ 16 mbar |

Tabelle 3 (Fortsetzung)

| Bsp.-Nr. | Formel | physikalische Eigenschaften |
|---|---|---|
| IV-16 | 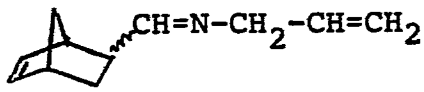 | Kp = 92-95°C/ 16 mbar |
| IV-17 | 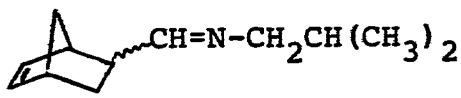 | Kp = 99-102°C/ 18 mbar |
| IV-18 | 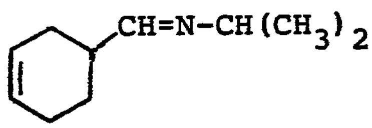 | Öl |
| IV-19 | 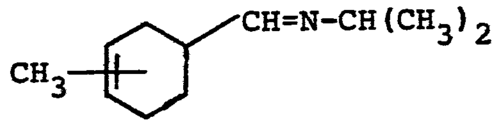 | Öl |
| IV-20 | 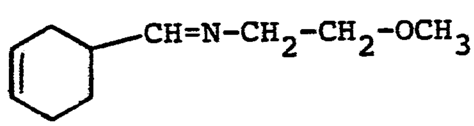 | Öl |

Tabelle 3 (Fortsetzung)

| Bsp.-Nr. | Formel | physikalische Eigenschaften |
|---|---|---|
| IV-21 | 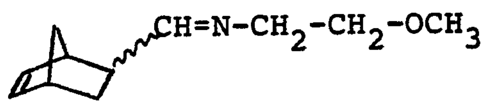 | Kp = 59-61°C/ 0,1 mbar |
| IV-22 | 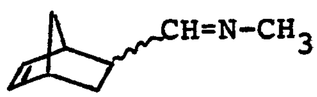 | Kp = 58-60°C/ 16 mbar |
| IV-23 | 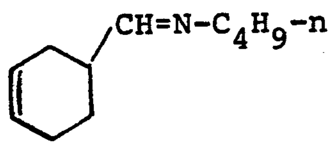 | Öl |
| IV-24 | 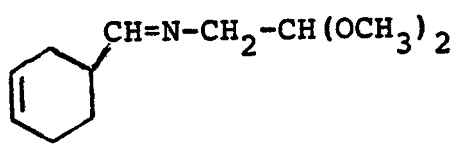 | Öl |

Anwendungsbeispiele:

In den folgenden Anwendungsbeispielen wurden die nachstehend aufgeführten Verbindungen als Vergleichssubstanzen eingesetzt:

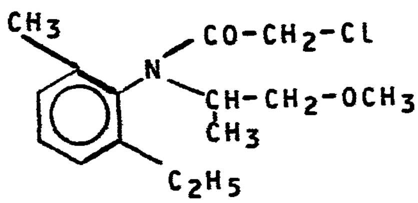 = (A)

2-Chlor-N-(2-ethyl-6-methyl-phenyl)-N-(1-methoxyprop-2-yl)-acetamid
(bekannt aus DE-OS 23 28 340)

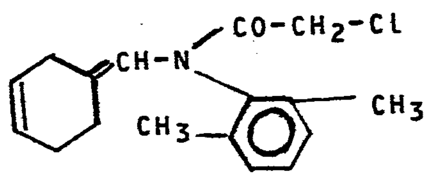 = (B)

N-(4-Cyclohex-1-enyliden-metheno)-2,6-dimethyl-chloracetanilid
(bekannt aus DE-OS 31 20 990).

## Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigte die erfindungsgemäße Verbindung gemäß Beispiel 1 bei der Bekämpfung von Panicum, Alopecurus und Avena in Weizen, Baumwolle und Zuckerrüben

0148393

eine deutlich bessere selektive herbizide Wirksamkeit als die Vergleichssubstanz (A).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigten die erfindungsgemäßen Stoffe gemäß Beispielen (1) und (14) eine wesentlich bessere selektive herbizide Wirksamkeit als die Vergleichssubstanz (B).

Patentansprüche

1. N-Chloracetyl-enamine der Formel

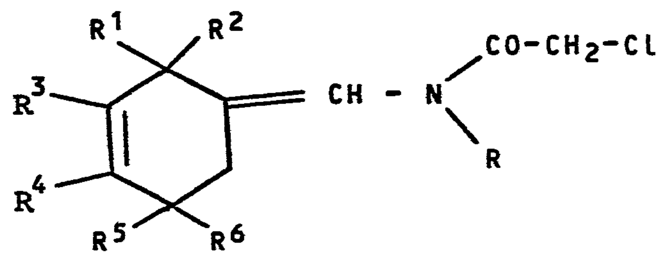

(I)

in welcher

R für Alkyl, Alkenyl, Alkinyl, Alkoxyalkyl, Dialkoxyalkyl, Alkylthioalkyl oder Halogenalkyl steht und

$R^1$, $R^2$, $R^3$, $R^4$, $R^5$ und $R^6$ unabhängig voneinander für Wasserstoff, Halogen, Alkyl, Alkenyl, Alkinyl, Alkoxy, Alkanoyloxy, Dialkylamino oder für gegebenenfalls substituiertes Aryl stehen, und außerdem

$R^1$ und $R^3$ oder
$R^1$ und $R^5$ und/oder
$R^3$ und $R^4$ oder
$R^4$ und $R^5$ jeweils gemeinsam für eine gegebenenfalls substituierte Alkylen-, Cycloalkylen-, Alkenylen-, Arylen- oder Etherbrücke, oder für eine Sauerstoffbrücke stehen können.

2. N-Chloracetyl-enamine der Formel (I), in denen

R für geradkettiges oder verzweigtes Alkyl mit

1 bis 10 Kohlenstoffatomen, Alkenyl mit 2 bis 10 Kohlenstoffatomen, Alkinyl mit 2 bis 10 Kohlenstoffatomen, geradkettiges oder verzweigtes Alkoxyalkyl mit 1 bis 6 Kohlenstoffatomen in der Alkoxygruppe und 1 bis 6 Kohlenstoffatomen im Alkylteil, Dialkoxyalkyl mit 1 bis 6 Kohlenstoffatomen in jeder Alkoxygruppe und 1 bis 6 Kohlenstoffatomen im Alkylteil, Alkylthioalkyl mit 1 bis 6 Kohlenstoffatomen im Alkylthioteil und 1 bis 6 Kohlenstoffatomen im Alkylteil oder für geradkettiges oder verzweigtes Halogenalkyl mit 1 bis 6 Kohlenstoffatomen und 1 bis 9 gleichen oder verschiedenen Halogenatomen steht und

$R^1$, $R^2$, $R^3$, $R^4$, $R^5$ und $R^6$ unabhängig voneinander vorzugsweise für Wasserstoff, Fluor, Chlor, Brom, Jod, geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Alkoxy mit 1 bis 4 Kohlenstoffatomen, Alkanoyloxy mit 1 bis 4 Kohlenstoffatomen in der Alkan-Gruppe, Dialkylamino mit 1 bis 4 Kohlenstoffatomen in jeder Alkyl-Gruppe oder für gegebenenfalls durch Halogen, Cyano, Nitro, Alkyl mit 1 bis 4 Kohlenstoffatomen und/oder Alkoxy mit 1 bis 4 Kohlenstoffatomen substituiertes Aryl mit 6 bis 10 Kohlenstoffatomen stehen, und außerdem

$R^1$ und $R^3$ oder
$R^1$ und $R^5$ und/oder
$R^3$ und $R^4$ oder
$R^4$ und $R^5$ jeweils gemeinsam für eine gegebenenfalls durch Halogen, Alkyl mit 1 bis 4 Kohlenstoffatomen und/oder Alkoxy mit 1 bis 4 Kohlenstoffatomen substituierte Alkylenbrücke mit 1 bis 6 Kohlenstoffatomen, eine gegebenenfalls durch Halogen, Alkyl mit 1 bis 4 Kohlenstoffatomen und/oder Alkoxy mit 1 bis 4 Kohlenstoffatomen substituierte Cycloalkylenbrücke mit 4 bis 8 Kohlenstoffatomen, für eine gegebenenfalls durch Halogen, Alkyl mit 1 bis 4 Kohlenstoffatomen und/oder Alkoxy mit 1 bis 4 Kohlenstoffatomen substituierte Alkenylenbrücke mit 2 bis 6 Kohlenstoffatomen, eine gegebenenfalls durch Halogen, Alkyl mit 1 bis 4 Kohlenstoffatomen und/oder Alkoxy mit 1 bis 4 Kohlenstoffatomen substituierte Arylenbrücke mit 6 bis 10 Kohlenstoffatomen oder für eine gegebenenfalls durch Halogen, Alkyl mit 1 bis 4 Kohlenstoffatomen und/oder Alkoxy mit 1 bis 4 Kohlenstoffatomen substituierte Etherbrücke mit 2 bis 6 Kohlenstoffatomen, oder für eine Sauerstoffbrücke stehen.

3. Verfahren zur Herstellung von N-Chloracetyl-enaminen der Formel

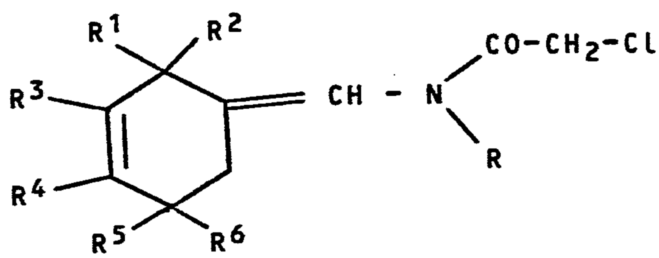

(I)

 

in welcher

R für Alkyl, Alkenyl, Alkinyl, Alkoxyalkyl, Dialkoxyalkyl, Alkylthioalkyl oder Halogenalkyl steht und

$R^1$, $R^2$, $R^3$, $R^4$, $R^5$ und $R^6$ unabhängig voneinander für Wasserstoff, Halogen, Alkyl, Alkenyl, Alkinyl, Alkoxy, Alkanoyloxy, Dialkylamino oder für gegebenenfalls substituiertes Aryl stehen, und außerdem

$R^1$ und $R^3$ oder
$R^1$ und $R^5$ und/oder
$R^3$ und $R^4$ oder
$R^4$ und $R^5$ jeweils gemeinsam für eine gegebenenfalls substituierte Alkylen-, Cycloalkylen-, Alkenylen-, Arylen- oder Etherbrücke, oder für eine Sauerstoffbrücke stehen können,

dadurch gekennzeichnet, daß man Aldehyde der Formel

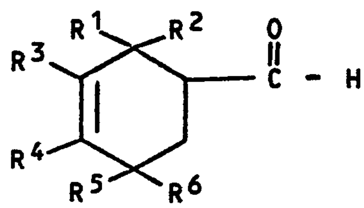 (II)

in welcher

$R^1$, $R^2$, $R^3$, $R^4$, $R^5$ und $R^6$ die oben angegebene Bedeutung haben,

zunächst in einer ersten Stufe mit Aminen der Formel

$$R - NH_2 \qquad (III)$$

in welcher

R die oben angegebene Bedeutung hat,

gegebenenfalls in Gegenwart eines Verdünnungsmittels und gegebenenfalls in Gegenwart eines Katalysators umsetzt und die so erhaltenen Azomethine der Formel

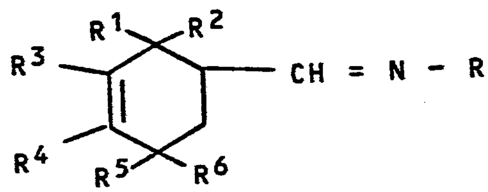

(IV)

in welcher

R, $R^1$, $R^2$, $R^3$, $R^4$, $R^5$ und $R^6$ die oben angegebene Bedeutung haben,

in einer 2. Stufe mit Chloracetylchlorid, gegebenenfalls in Gegenwart eines Verdünnungsmitttels und gegebenenfalls in Gegenwart eines Säurebindemittels umsetzt.

4. Herbizide Mittel, gekennzeichnet durch einen Gehalt an mindestens einem N-Chloracetyl-enamin der Formel (I).

5. Verfahren zur Bekämfpung von Unkräutern, dadurch gekennzeichnet, daß man N-Chloracetyl-enamine der

0148393

Formel (I) auf die Unkräuter und/oder deren Lebensraum ausbringt.

6. Verwendung von N-Chloracetyl-enaminen der Formel (I) zur Bekämpfung von Unkräutern.

7. Verfahren zur Herstellung von herbiziden Mitteln, dadurch gekennzeichnet, daß man N-Chloracetyl-enamine der Formel (I) mit Streckmitteln und/oder oberflächenaktiven Stoffen vermischt.

8. Azomethine der Formel

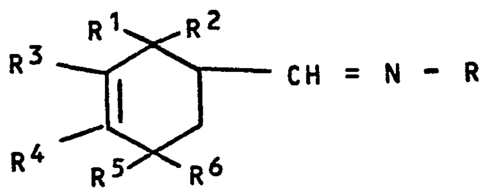

(IV)

in welcher

R für Alkyl, Alkenyl, Alkinyl, Alkoxyalkyl, Dialkoxyalkyl, Alkylthioalkyl oder Halogenalkyl steht und

$R^1$, $R^2$, $R^3$, $R^4$, $R^5$ und $R^6$ unabhängig voneinander für Wasserstoff, Halogen, Alkyl, Alkenyl, Alkinyl, Alkoxy, Alkanoyloxy, Dialkylamino oder für gegebenenfalls substituiertes Aryl stehen, und außerdem

$R^1$ und $R^3$ oder

0148393

$R^1$ und $R^5$ und/oder
$R^3$ und $R^4$ oder
$R^4$ und $R^5$ jeweils gemeinsam für eine gegebenenfalls substituierte Alkylen-, Cycloalkylen-, Alkenylen-, Arylen- oder Etherbrücke, oder für eine Sauerstoffbrücke stehen können.

9. N-Chloracetyl-enamin der Formel

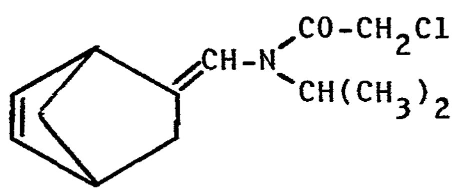

.

10. N-Chloracetyl-enamin der Formel

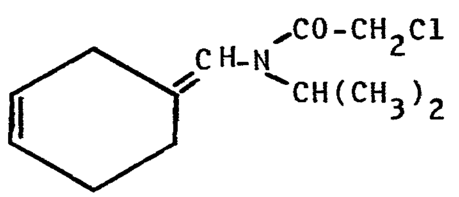

.

Europäisches Patentamt

**EUROPÄISCHER RECHERCHENBERICHT**

0148393 Nummer der Anmeldung

EP 84 11 4311

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| Y | DE-A-2 526 868 (CIBA-GEIGY AG)<br>* Ansprüche *<br>--- | 1,3-8 | C 07 C 103/37<br>103/38<br>119/06<br>A 01 N 37/18 |
| D,Y | EP-A-0 065 712 (BAYER AG)<br>* Ansprüche *<br>--- | 1,3-8 | |
| X | DD-A- 63 485 (E. PROFFT)<br>* Insgesamt *<br>--- | 8 | |
| X | JOURNAL OF THE CHEMICAL SOCIETY, CHEMICAL COMMUNICATIONS, Band D, Nr. 19, 8. Oktober, 1969, Seiten 1151-1152, J.C. LEFFINGWELL et al.: "A New Synthetic Method for the Preparation of Aromatic Aldehydes, Ketones, and Schiff Bases".<br>* Seite 1152 *<br>--- | 8 | RECHERCHIERTE SACHGEBIETE (Int. Cl.4)<br>C 07 C 103/00<br>C 07 C 119/00<br>A 01 N 37/00 |
| X | ANGEWANDTE CHEMIE, INTERNATIONAL EDITION, Band 11, Nr. 11, November 1972, Seiten 1011-1012, H. HAGEMANN et al.: "Cyclization of Azomethines by N-Chlorocarbonyl Isocyanate".<br>* Seiten 1011-1012 *<br>--- -/- | 8 | |

*Der vorliegende Recherchenbericht wurde für alle Patentansprüche erstellt.*

| Recherchenort | Abschlußdatum der Recherche | Prüfer |
|---|---|---|
| DEN HAAG | 12-03-1985 | MOREAU J.M. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

0148393

Nummer der Anmeldung

EP 84 11 4311
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 64, Nr. 8, 11. April, 1966, Spalte 11096-Spalte 11097, COLUMBUS, OHIO, (US) P. KAIKARIS: "Condensation of 3-cyclohexenyl aldehydes with ethyl chloroacetate and primary amines, and preparation of hydrazothiazolinones". & Probl. Organ. Sinteza, Akad. Nauk SSSR, Otd. Obshch. i Tekhn. Khim. 1965, 84-9. ----- | 8 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 12-03-1985 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument